# EUROPÄISCHE PATENTSCHRIFT

(11) **EP 4 427 015 B1**
(45) Veröffentlichungstag und Bekanntmachung des Hinweises auf die Patenterteilung: **28.01.2026**
(21) Anmeldenummer: 22809061.9
(22) Anmeldetag: 24.10.2022
(51) Int. Cl.: G01N 1/22, G01N 27/18

(54) **SENSOR ZUR ERFASSUNG MINDESTENS EINER EIGENSCHAFT EINES FLUIDEN MEDIUMS IN EINEM MESSRAUM**
SENSOR FOR DETECTING AT LEAST ONE PROPERTY OF A FLUID MEDIUM IN A MEASUREMENT CHAMBER
CAPTEUR DE DÉTECTION D'AU MOINS UNE PROPRIÉTÉ D'UN MILIEU FLUIDE DANS UNE CHAMBRE DE MESURE

(30) Priorität: 04.11.2021 DE 102021212418
(43) Veröffentlichungstag der Anmeldung: 11.09.2024
(73) Patentinhaber: Robert Bosch GmbH, 70442 Stuttgart (DE)
(72) Erfinder: LANG, Tobias, 70197 Stuttgart (DE); ALT, Christian, 70806 Kornwestheim (DE); BRAUER, Ingo, 71665 Vaihingen (DE); HUEFTLE, Gerhard, 71546 Aspach (DE); HERRMANN, Reinhold, 70469 Stuttgart (DE)
(86) Internationale Anmeldenummer: PCT/EP2022/079620
(87) Internationale Veröffentlichungsnummer: WO 2023/078716

(56) Entgegenhaltungen:
- EP-A2- 3 546 955
- DE-T2- 60 002 044
- DE-T5- 112014 002 928

## Beschreibung

### Stand der Technik

Aus dem Stand der Technik ist eine Vielzahl von Sensoren, Sensorelementen und Verfahren zur Erfassung mindestens einer Eigenschaft eines fluiden Mediums in einem Messraum bekannt. Dabei kann es sich grundsätzlich um beliebige Eigenschaften eines gasförmigen oder flüssigen fluiden Mediums handeln, wobei eine oder mehrere Eigenschaften erfasst werden können. Die Erfindung wird im Folgenden, ohne Beschränkung weiterer Ausführungsformen und Anwendungen, insbesondere unter Bezugnahme auf Sensorelemente zur Erfassung eines Gases, insbesondere eines H₂-Anteils in einem Messgas, beschrieben.

Sensorelemente der hier beschriebenen Art finden Anwendung in einer Vielzahl von Gebieten, beispielsweise in der Automobiltechnik, der Verfahrenstechnik, der Chemie und dem Maschinenbau, insbesondere zur Bestimmung von Gaskonzentrationen. So spielt beispielsweise die Bestimmung von Wasserstoffkonzentrationen, beispielsweise in einem Luft-Wasserstoff-Gemisch, bei der Anwendung von Wasserstoff-Brennstoffzellen-Systemen eine große Rolle. Hierbei sind auch sicherheitsrelevante Anwendungen zu nennen. Ein Luft-Wasserstoff-Gemisch wird etwa bei einem Wasserstoffanteil von 4 % zündungsfähig. Sensorelemente zur Erfassung von Wasserstoff können beispielsweise in Wasserstoff-Brennstoffzellenfahrzeugen zum Einsatz kommen, um beispielsweise aufgrund von Beschädigung oder Defekt austretenden Wasserstoff zu detektieren und, durch eine Kopplung an entsprechende Systeme, Warnsignale und/oder Schutzmaßnahmen auszulösen. Daher werden pro Brennstoffzellenfahrzeug mehrere Wasserstoffsensoren benötigt, die entweder im Abgasstrang angebracht werden (exhaust) oder unter atmosphärischen Bedingungen arbeiten (ambient).

Für derartige Wasserstoffsensoren kann man auf eine Vielzahl von Messprinzipien zurückgreifen. Dazu gehören u.a. folgende Messprinzipien: Wärmeleitung, katalytischer Pellistor, elektrochemische Zelle, halbleitendes Metalloxid, Chemiresistor, Feldeffekt Transistor. Sensorelemente zum Messen einer Wärmeleitfähigkeit sind beispielsweise aus der DE 10 2005 058 832 A1 und DE 10 2014 202 169 A1 bekannt.

DE112014002928T5 offenbart einen Gassensor in einem Abgasrohr, umfassend ein Wärmeleitfähigkeits-Sensorelement, ein Abzweigungselement mit Strömungsrohr und Innenraum, sowie eine Blende.

Trotz der Vorteile der aus dem Stand der Technik bekannten Sensorelemente zur Erfassung mindestens einer Eigenschaft eines fluiden Mediums beinhalten diese noch Verbesserungspotenzial. Soll die Wärmeleitfähigkeit mit einem Messelement bestehend aus einer dünnen Membran gemessen werden, ist eine Konvektion des Abgases vorbei an der Membran zu vermeiden, da dies eine Verfälschung des Messsignals bedeutet. Stattdessen muss das fluide Medium an der Membran möglichst in Ruhe sein. Im Abgasstrang einer Brennstoffzelle liegen jedoch Strömungsgeschwindigkeiten des Gases von bis zu 100 m/s vor. Zusätzlich wird viel Wasser produziert und liegt teilweise als Tropfen vor, die ebenfalls die Messung verfälschen können.

### Offenbarung der Erfindung

Im Rahmen der vorliegenden Erfindung wird daher ein Sensor zur Erfassung mindestens einer Eigenschaft eines fluiden Mediums in einem Messraum vorgeschlagen, welches die Nachteile bekannter Sensoren zur Erfassung mindestens einer Eigenschaft eines fluiden Mediums in einem Messraum zumindest weitgehend vermeidet, und das ausreichend Empfindlichkeit, Messbereich, Reaktionszeit und Selektivität bezüglich der Anforderungen in der Automobiltechnik bietet.

Ein erfindungsgemäßer Sensor zur Erfassung mindestens einer Eigenschaft eines fluiden Mediums in mindestens einem Messraum, insbesondere zur Erfassung eines H₂-Anteils in einem Messgas, umfasst mindestens ein Sensorelement, das zum Erfassen einer Wärmeleitfähigkeit des fluiden Mediums und zum Ausgeben eines Messsignals ausgebildet ist.

Bei dem Messraum kann es sich grundsätzlich um einen beliebigen, offenen oder geschlossenen, Raum handeln, in welchem das fluide Medium, insbesondere das Messgas, aufgenommen ist, und/oder welcher von dem fluiden Medium, insbesondere dem Messgas, durchströmt wird.

Das Sensorelement ist beispielsweise als Sensorchip mit einer beheizbaren Messmembran ausgebildet. Beispielsweise umfasst der Sensorchip eine Chipoberfläche. Die Chipoberfläche weist eine mit dem fluiden Medium beaufschlagbare Messoberfläche und eine Festlandsoberfläche auf. Auf der Messoberfläche sind Leiterbahnen einer Sensorschaltung mit mindestens einem Heizelement aufgebracht. Auf der Festlandsoberfläche sind Leiterbahnen der Sensorschaltung mit mindestens einem Temperaturfühler aufgebracht.

Der Sensorchip kann auf einem Sockel, insbesondere einem Glassockel oder einem Siliziumsockel angeordnet sein. Eine Verbindung zwischen dem Sensorchip und dem Sockel kann mittels anodischen Bondens realisiert werden.

In dem Sockel kann auch ein Zugangskanal ausgebildet werden. Beispielsweise wird der Zugangskanal in Form einer Bohrung realisiert. Der Zugangskanal kann durch Ritz- oder Sägeprozesse oder durch Ätzprozesse hergestellt werden. Bei einer derartigen Ausführung ist es möglich, auf der dem Sockel zugewandten Seite der Messoberfläche, d.h. der Unterseite der Messoberfläche, einen Raum zu bilden. Dieser Raum kann beispielsweise ähnlich einer Kaverne ausgebildet sein.

Unter einer Membran kann im Rahmen der vorliegenden Erfindung eine dünne Struktur verstanden werden, die wie eine Haut oder Folie im Verhältnis zu ihrer Dicke eine große flächige Ausdehnung hat.

Der Sensor umfasst weiterhin ein Abzweigungselement, das einen Innenraum definiert. Das Abzweigungselement ist zum Abzweigen eines Teils des fluiden Mediums aus dem Messraum in den Innenraum ausgebildet ist.

Der Sensor umfasst weiterhin mindestens eine Blende. Das Sensorelement ist mit dem Innenraum mittels der Blende fluidverbunden.

Unter einer Blende kann im Rahmen der vorliegenden Erfindung ein Bauteil verstanden werden, das eine Querschnittsverengung bewirkt. Zu diesem Zweck weist die Blende eine Öffnung auf, so dass ein fluides Medium durch diese hindurchströmen kann. Die Öffnung weist dabei eine deutlich geringere Querschnittsfläche als an die Blende angrenzende Bereiche auf.

Bei dem erfindungsgemäßen Sensor greift das Abzweigungselement einen Teil des fluiden Mediums ab, das in einer Strömungsbewegung sein kann, und führt dieses in Richtung des Sensorelements ab. Das fluide Medium hat bei Durchtritt durch die Blende konvektive und diffusive Anteile. Auf diesem Strömungspfad wird durch eine oder mehrere Blenden die konvektive Strömung beruhigt.

Die Blende kann zum Verringern von Turbulenzen des fluiden Mediums ausgebildet sein. Dadurch wird die Strömung des fluiden Mediums beruhigt.

Die Blende kann zum Verringern von konvektiven Teilen des fluiden Mediums ausgebildet sein. Dadurch wird die Strömung des fluiden Mediums beruhigt und insbesondere konvektive Anteile verringert, so dass eine Verfälschung des Messsignals deutlich verringert wird.

Die Blende kann derart ausgebildet sein, dass das fluide Medium zu dem Sensorelement im Wesentlichen mittels Diffusion gelangt. Dadurch ist die Bewegung des fluiden Mediums an dem Sensorelement so gering, dass durch den verbleibenden konvektiven Anteil kein Signalfehler mehr entsteht. Entsprechend gelangt das fluide Medium zu dem Sensorelement überwiegend durch Diffusion. Der Ausdruck "im Wesentlichen mittels Diffusion" kann dabei so verstanden werden, dass die Geschwindigkeit des fluiden Mediums aus dem Messraum so stark reduziert wird, dass sie im Bereich am Sensorelement nur noch 0,2%, bevorzugt nur noch 0,1% und noch bevorzugter nur noch 0,05% der Geschwindigkeit im Messraum beträgt.

Die Blende kann zum im Wesentlichen Verhindern einer Wärmeübertagung von dem Sensorelement auf das fluide Medium ausgebildet sein. Dadurch wird eine Verfälschung der Wärmeleitfähigkeitsmessung verhindert und durch den verbleibenden konvektiven Anteil entsteht kein Signalfehler mehr. Der Ausdruck "im Wesentlichen Verhindern einer Wärmeübertagung von dem Sensorelement auf das fluide Medium" kann dabei so verstanden werden, dass eine Wärmeübertragung nur unwesentlich ist. Mit anderen Worten ist die Strömung am Sensorelement so gering, dass eine Wärmeabfuhr von diesem durch einen konvektiven Anteil des fluiden Mediums so gering ist, dass die Messung der Wärmeleitfähigkeit des fluiden Mediums nur unwesentlich beeinflusst wird, der Messfehler bezogen auf das maximale Signal des Sensorelementes somit nicht größer als 5 %, bevorzugt nicht größer als 3 % und noch bevorzugter nicht größer als 2 % ist. Es versteht sich jedoch, dass eine Wärmeübertragung auch ohne Konvektion stattfindet, nämlich durch Wärmeleitung, da dies das Messprinzip des Sensors ist.

Die Blende bzw. die Blenden ist bzw. sind dabei bevorzugt derart gestaltet, dass diese mit ihrer Öffnungsfläche die Querschnittsfläche jeweils auf 1/10 des Strömungsquerschnittes des angrenzenden Raums reduzieren.

Das Abzweigungselement ist zum Verringern einer Strömungsgeschwindigkeit des fluiden Mediums beim Abzweigen aus dem Messraum in den Innenraum ausgebildet ist. So greift das Abzweigungselement einen Teil des fluiden Mediums ab, das in einer Strömungsbewegung sein kann, und führt dieses möglichst schnell bzw. über kurze Strecken in Richtung des Sensorelements ab. Dadurch wird der Austausch des fluiden Mediums und die Ansprechzeit des Sensorelements erhöht.

Das Abzweigungselement kann derart ausgebildet sein, dass der Teil des fluiden Mediums, der aus dem Messraum in den Innenraum abzweigbar ist, nicht mehr als 10 %, bevorzugt nicht mehr als 5 % und noch bevorzugter nicht mehr als 3 % eines Volumenstroms des fluiden Mediums in dem Messraum ist. Dadurch fließt nur eine kleiner, aber repräsentativer Anteil der Hauptströmung des fluiden Mediums durch den Sensor und eventuelle vorhandene Wassertropfen oder andere feste Partikel können aufgrund ihrer Masseträgheit nicht zum Sensorelement gelangen.

Der Sensor kann weiterhin mehrere Blenden umfassen, wobei die Blenden in einer Strömungsrichtung von dem Messraum zu dem Sensorelement gesehen derart hintereinander angeordnet sind, dass konvektive Anteile des fluiden Medium in der Strömungsrichtung gesehen abnehmen. Durch Hintereinanderschalten mehrere Blenden kann die Strömung des fluiden Mediums so weit beruhigt werden, dass die Bewegung des fluiden Mediums an dem Sensorelement so gering ist, dass durch den verbleibenden konvektiven Anteil kein Signalfehler mehr entsteht.

Das Abzweigungselement ist als eine Venturidüse mit einem Abnahmeroh ausgebildet. Durch die Umsetzung als Venturidüse können große Wassertropfen bedingt durch ihre Masseträgheit durch den Sensor hindurchfliegen und werden so von dem Sensorelement ferngehalten. Sehr kleine Tropfen können ggf. der Strömung folgen, sind dann aber aufgrund ihrer geringen Größe für die Messung unkritisch.

Unter einer Venturidüse ist im Rahmen der vorliegenden Erfindung ein Bauteil in Form eines Rohrstücks zu verstehen, das im kennzeichnenden Teil des Anspruchs 1 definiert wird. Das Wirkprinzip beruht darauf, dass wenn ein Fluid durch eine Venturi-Düse fließt, an der engsten Stelle des Rohres der dynamische Druck (Staudruck) maximal und der hydrostatische Druck minimal ist. Die Geschwindigkeit des Fluids steigt im Verhältnis der Querschnitte beim Einströmen in den engeren Teil an, weil im ganzen Rohr dieselbe Masse pro Zeit durchfließt (Kontinuitätsgesetz). Dadurch sinkt der Druck im Abnahmerohr, das sich im engen Teil befindet. Damit entsteht ein Differenzdruck, der in Messgeräten oder zum Ansaugen von Flüssigkeiten oder Gasen benutzt werden kann. Die Druckdifferenz ist bei idealen Flüssigkeiten (inkompressibel und reibungsfrei) durch die Bernoulli-Gleichung gegeben. Bei idealen Gasen gilt die erweiterte Bernoulli-Gleichung.

Das Abzweigungselement kann zum im Wesentlichen Verhindern eines Zutritts von Flüssigkeitstropfen und/oder Partikeln zu dem Sensorelement ausgestaltet sein. Der Ausdruck "zum im Wesentlichen Verhindern" ist dabei so zu verstehen, dass keine Tropfen und/oder Partikel oberhalb einer vorbestimmten Größe zu dem Sensorelement gelangen. Nur sehr kleine Tropfen oder Partikel, d.h. unterhalb der vorbestimmten Größe, können ggf. der Strömung folgen, sind dann aber aufgrund ihrer geringen Größe für die Messung unkritisch.

Der Messraum kann ein Strömungsrohr sein, insbesondere ein Abgasrohr einer Brennstoffzelle. Entsprechend eignet sich der Sensor auch für Bereiche, in denen vergleichsweise hohe Strömungsgeschwindigkeiten vorliegen.

### Kurze Beschreibung der Zeichnungen Weitere Einzelheiten und Merkmale der Erfindung ergeben sich aus der nachfolgenden Beschreibung bevorzugter Ausführungsbeispiele, welche in den Figuren schematisch dargestellt sind.

Es zeigt:

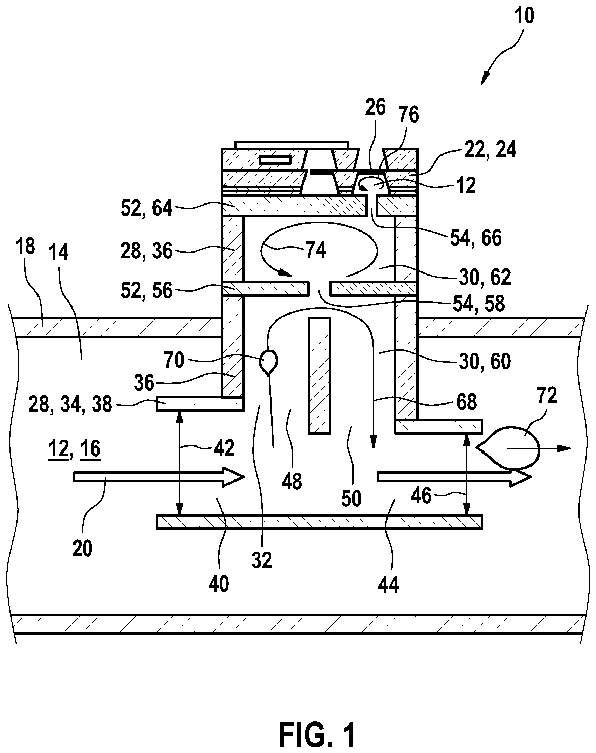

Figur 1 eine Querschnittsansicht eines Sensors gemäß einer Ausführungsform der vorliegenden Erfindung.

### Ausführungsformen der Erfindung

Figur 1 ist eine Querschnittsansicht eines Sensors 10 zur Erfassung mindestens einer Eigenschaft eines fluiden Mediums 12 in mindestens einem Messraum 14, insbesondere zur Erfassung eines H₂-Anteils in einem Messgas 16. Der Sensor 10 kann insbesondere zum Einsatz in einem Wasserstoff-Brennstoffzellenfahrzeug eingerichtet sein. Auch andere Anwendungen sind jedoch möglich. Der Sensor 10 kann insbesondere ein oder mehrere in den Figuren nicht dargestellte, weitere Funktionselemente umfassen, wie beispielsweise Elektroden, Elektrodenzuleitungen und Kontakte, mehrere Schichten oder andere Elemente. Entsprechend kann der Sensor 10 im Abgasstrang des Wasserstoff-Brennstoffzellenfahrzeugs angebracht werden (exhaust) oder unter atmosphärischen Bedingungen arbeiten (ambient). Folglich kann es sich bei dem Messraum um einen Abgasstrang oder Innenraum des Wasserstoff-Brennstoffzellenfahrzeugs handeln. Bei der dargestellten Ausführungsform handelt es bei dem Messraum 14 um ein Strömungsrohr in Form eines Abgasrohrs 18.

Der Sensor 10 ist an dem Abgasrohr 18 angebracht, wie beispielsweise verschraubt, eingesteckt oder verschweißt. In dem Abgasrohr 18 strömt das fluide Medium in Form eines Gases oder einer Gasmischung, die Wasserstoff enthält, in einer Hauptströmungsrichtung 20. Die Strömungsgeschwindigkeit kann dabei bis zu 100 m/s betragen.

Der Sensor 10 weist ein Sensorelement 22 auf. Das Sensorelement 22 ist zum Erfassen einer Wärmeleitfähigkeit des fluiden Mediums und zum Ausgeben eines Messsignals ausgebildet ist. Zu diesem Zweck ist das Sensorelement 22 als Sensorchip 24 mit einer beheizbaren Membran 26 ausgebildet. Das Beheizen der Membran 26 erfolgt mittels eines nicht näher gezeigten Heizelements, das auf der Membran 26 angeordnet ist. Das Ausgeben des Messsignals erfolgt mittels einer nicht näher gezeigten Temperaturfühlerleiterbahn, die auf einer Festlandsoberfläche des Sensorchips 24 angeordnet ist.

Der Sensor 10 weist weiterhin ein Abzweigungselement 28 auf. Das Abzweigungselement 28 definiert einen Innenraum 30. Das Abzweigungselement 28 ist zum Abzweigen eines Teils des fluiden Mediums 12 aus dem Messraum 14 in den Innenraum 30 ausgebildet. Zu diesem Zweck weist das Abzweigungselement 28 mindestens eine Öffnung 32 auf. Weiterhin ist das Abzweigungselement 28 mit dem Messraum 14 verbindbar. Figur 1 zeigt dabei das Abzweigungselement 28 im verbundenen Zustand. Das Abzweigungselement 28 ist zum Verringern einer Strömungsgeschwindigkeit des fluiden Mediums 12 beim Abzweigen aus dem Messraum 14 in den Innenraum ausgebildet. Mit anderen Worten ist das Abzweigungselement 28 so gestaltet, dass sich eine Strömungsgeschwindigkeit des fluiden Mediums 12 beim Eindringen in den Innenraum 30 verringert. Weiterhin ist das Abzweigungselement 28 derart ausgebildet, dass der Teil des fluiden Mediums 12, der aus dem Messraum 14 in den Innenraum 30 abzweigbar ist, nicht mehr als 10 %, bevorzugt nicht mehr als 5 % und noch bevorzugter nicht mehr als 3 % eines Volumenstroms des fluiden Mediums 12 in dem Messraum 14 ist. Dadurch fließt nur eine kleiner, aber repräsentativer Anteil der Hauptströmung des fluiden Mediums 12 durch den Sensor 10 und eventuell vorhandene Wassertropfen oder andere feste Partikel können aufgrund ihrer Masseträgheit nicht zum Sensorelement 22 gelangen. Mit anderen Worten, fließt der größte Teil des fluiden Mediums aus dem Messraum 14 am Sensor 10 vorbei und von dem Teil der in das Abzweigungselement 28 eintritt, fließt nur ein Teil in den Innenraum 30.

Wie in Figur 1 gezeigt, ist das Abzweigungselement 28 als Venturidüse 34 mit einem Abnahmerohr 36 ausgebildet. So weist das Abzweigungselement 28 ein Strömungsrohr 38 auf, das sich parallel zur Hauptströmungsrichtung 20 des fluiden Mediums im Abgasrohr 18 erstreckt. Das Strömungsrohr 38 ist dabei von dem fluiden Medium 12 durchströmbar. Das Strömungsrohr 38 weist dabei zumindest einen ersten Rohrabschnitt 40 mit einer ersten Querschnittsfläche 42 und einen zweiten Rohrabschnitt 44 mit einer zweiten Querschnittsfläche 46 auf. Der erste Rohrabschnitt 40 ist der Hauptströmungsrichtung 20 zugewandt und der zweite Rohrabschnitt 44 ist der Hauptströmungsrichtung 20 abgewandt. Mit anderen Worten befindet sich der erste Rohrabschnitt 40 am stromaufwärtigen Ende des Strömungsrohrs 38 und der zweite Rohrabschnitt 44 am stromabwärtigen Ende des Strömungsrohrs 38. Die erste Querschnittsfläche 42 ist größer als die zweite Querschnittsfläche 46. Es versteht sich, dass das Strömungsrohr 38 weitere Rohrabschnitte aufweisen kann. Beispielsweise kann das Strömungsrohr 38 einen dritten Rohrabschnitt auf, der sich weiter stromabwärts als der zweite Rohrabschnitt 44 befindet und eine dritte Querschnittsfläche aufweist. Die dritte Querschnittsfläche ist größer als die zweite Querschnittsfläche und kann beispielsweise gleich groß wie die erste Querschnittsfläche sein.

Das Abnahmerohr 36 definiert den Innenraum 30. Das Abnahmerohr 36 ist bei dem gezeigten Ausführungsbeispiel im Wesentlichen senkrecht zu dem Strömungsrohr 38 orientiert. Es wird jedoch explizit betont, dass auch andere Orientierungen des Abnahmerohrs 36 möglich sind. So ist die Orientierung des Abnahmerohrs 36 relativ zu dem Strömungsrohr 38 für das Messprinzip des Sensors 10 nicht entscheidend.

Das Abnahmerohr 36 ist gebogen. Das Abnahmerohr 36 ist mit dem Strömungsrohr 38 im Bereich der Querschnittsverengung des Strömungsrohrs 38 verbunden. Das Abnahmerohr 36 ist U-förmig ausgebildet. So weist das Abnahmerohr 36 eine erste Öffnung 48, die mit dem ersten Rohrabschnitt 40 fluidverbunden ist, und eine zweite Öffnung 50, die mit dem zweiten Rohrabschnitt 44 fluidverbunden ist, auf.

Der Sensor 10 weist weiterhin mindestens eine Blende 52 auf. Das Sensorelement 22 ist mit dem Innenraum 30 mittels der Blende 52 fluidverbunden. Entsprechend ist das Sensorelement 22 nicht unmittelbar mit dem Messraum 14 verbunden, sondern über den Innenraum 30 des Abzweigungselements 28. Die Blende 52 ist zum Verringern von Turbulenzen des fluiden Mediums 12 ausgebildet. Die Blende 52 ist zum Verringern von konvektiven Teilen des fluiden Mediums 12 ausgebildet. Insbesondere ist die Blende 52 derart ausgebildet, dass das fluide Medium 12 zu dem Sensorelement 22 im Wesentlichen mittels Diffusion gelangt. Weiterhin ist die Blende 52 zum im Wesentlichen Verhindern einer Wärmeübertagung von dem Sensorelement 22 auf das fluide Medium 12 ausgebildet ist. Zu diesem Zweck ist die Blende 52 mit einer Blendenöffnung 54 ausgebildet, die einen vergleichsweise kleinen Querschnitt aufweist.

Wie in Figur 1 gezeigt, weist der Sensor 10 bei der gezeigten Ausführungsform mehrere Blenden 52 auf. Dabei sind die Blenden 52 in einer Strömungsrichtung von dem Messraum 14 zu dem Sensorelement 22 gesehen derart hintereinander angeordnet sind, dass konvektive Anteile des fluiden Mediums 12 in der Strömungsrichtung gesehen abnehmen. Genauer weist der Sensor 10 bei der gezeigten Ausführungsform eine erste Blende 56 mit einer ersten Blendenöffnung 58 auf, die den Innenraum 30 in einen ersten Innenraumabschnitt 60 und einen zweiten Innenraumabschnitt 62 unterteilt. Der erste Innenraumabschnitt 60 grenzt dabei an das Strömungsrohr 38 bzw. das Abgasrohr 18 an. Weiterhin weist der Sensor 10 eine zweite Blende 64 mit einer zweiten Blendenöffnung 66 auf. Die zweite Blende 64 ist von der ersten Blende 56 beabstandet und grenzt an den zweiten Innenraumabschnitt 62. Über die zweite Blendenöffnung 66 ist das Sensorelement 22 mit dem zweiten Innenraumabschnitt 62 fluidverbunden. Die zweite Blendenöffnung 66 weist eine kleinere Querschnittsfläche als die erste Blendenöffnung 54 auf.

Die Betriebsweise des Sensors 10 wird nachstehend beschrieben. Das fluide Medium 12 strömt in dem Abgasrohr 18, das den Messraum 14 darstellt mit einer vergleichsweisen hohen Geschwindigkeit von bis zu 100 m/s. Dabei durchströmt ein Teil des fluiden Mediums auch das Strömungsrohr 38 des Abzweigungselements 28. Wie oben beschrieben ist das Abnahmerohr 36 im Bereich des Innenraums 30 und genauer des ersten Innenraumabschnitts 60 U-förmig ausgebildet. Dadurch wird das fluide Medium 12 aus dem Messraum 14 bzw. dem Strömungsrohr 38 teilweise abgezweigt und gelangt durch die erste Öffnung 48 so in den Innenraum 30 bzw. zunächst in den ersten Innenraumabschnitt 60, wobei ein Teil des abgezweigten fluiden Mediums 12 um etwa 180° umgelenkt und durch die zweite Öffnung 50 wieder in das Strömungsrohr 38 austritt, wie durch einen Pfeil 68 angedeutet ist. Kleinere Wassertropfen 70 oder Partikel können dieser Strömungsumlenkung folgen, treten aber ebenfalls wieder in das Strömungsrohr 38 aus oder sind ohne Einfluss auf das Messsignal des Sensorelements 22. Größere Wassertropfen 72 oder Partikel können der Strömungsumlenkung nicht folgen und strömen mit dem fluiden Medium 12 in der Hauptströmungsrichtung 20 weiter durch das Strömungsrohr 38 hindurch. Auf diese Weise ist das Abzweigungselement 28 ausgestaltet im Wesentlichen einen Zutritt von Tropfen und/oder Partikeln zu dem Sensorelement 22 zu verhindern.

Ein anderer Teil des abgezweigten fluiden Mediums 12 gelangt durch die erste Blendenöffnung 58 der ersten Blende 56 in den zweiten Innenraumabschnitt 62 mittels Diffusion und Konvektion, wie durch einen Pfeil 74 angedeutet ist. Aus dem zweiten Innenraumabschnitt 62 gelangt das fluide Medium 12 zu dem Sensorelement durch die zweite Blendenöffnung 66 der zweiten Blende 64 vorwiegend mittels Diffusion, wie durch einen Pfeil 76 angedeutet ist. Konvektive Strömungsanteile des fluiden Mediums 12 bei Kontakt mit der Membran 26 sind dabei nicht oder nur unwesentlich vorhanden, so dass das Sensorelement 22 eine unverfälschte Messung der Wärmeleitfähigkeit des fluiden Mediums 12 durchführen kann. Entsprechend findet eine sukzessive Verringerung der Strömungsgeschwindigkeit des fluiden Mediums 12 ausgehend von dem Messraum 14, über den ersten Innenraumabschnitt 60 und den zweiten Innenraumabschnitt 62 und schließlich in dem an das Sensorelement 22 angrenzenden Bereich statt.

## Patentansprüche

1. Sensor (10) zur Erfassung mindestens einer Eigenschaft eines fluiden Mediums (12) in einem Messraum (14), insbesondere zur Erfassung eines H₂-Anteils in einem Messgas (16), umfassend:
mindestens ein Sensorelement (22), das zum Erfassen einer Wärmeleitfähigkeit des fluiden Mediums (12) und zum Ausgeben eines Messsignals ausgebildet ist, wobei der Sensor (10) weiterhin
ein Abzweigungselement (28), das einen Innenraum (30) definiert, wobei das Abzweigungselement (28) zum Abzweigen eines Teils des fluiden Mediums (12) aus dem Messraum (14) in den Innenraum (30) ausgebildet ist,
mindestens eine Blende (52, 56, 64), wobei das Sensorelement (22) mit dem Innenraum (30) mittels der Blende (52, 56, 64) fluidverbunden ist, und ein Abgasrohr (12), in dem das fluide Medium (12) in einer Hauptströmungsrichtung (20) strömt, aufweist, **dadurch gekennzeichnet, dass** das Abzweigungselement (28) als Venturidüse (34) mit einem U-förmigen Abnahmerohr (36) ausgebildet ist, wobei das Abzweigungselement (28) ein Strömungsrohr (38) aufweist, das sich parallel zu der Hauptströmungsrichtung des fluiden Mediums in dem Abgasrohr (18) erstreckt, wobei das Strömungsrohr (38) einen der Hauptströmungsrichtung (20) zugewandten ersten Rohrabschnitt (40) mit einer ersten Querschnittsfläche (42) und einen von der Hauptströmungsrichtung (20) abgewandten zweiten Rohrabschnitt (44) mit einer zweiten Querschnittsfläche (46) aufweist, wobei die erste Querschnittsfläche (42) größer als die zweite Querschnittsfläche (46) ist, wobei das Abnahmerohr (36) mit dem Strömungsrohr (38) im Bereich einer Querschnittsverengung des Strömungsrohres (38) verbunden ist, wobei das Abnahmerohr (36) eine erste Öffnung, die mit dem ersten Rohrabschnitt (40) fluidverbunden ist, und eine zweite Öffnung, die mit dem zweiten Rohrabschnitt (44) fluidverbunden ist, aufweist.

2. Sensor (10) nach dem vorhergehenden Anspruch, wobei die Blende (52, 56, 64) zum Verringern von Turbulenzen des fluiden Mediums (12) ausgebildet ist.

3. Sensor (10) nach einem der vorhergehenden Ansprüche, wobei die Blende (52, 56, 64) zum Verringern von konvektiven Teilen des fluiden Mediums (12) ausgebildet ist.

4. Sensor (10) nach einem der vorhergehenden Ansprüche, wobei die Blende (52, 56, 64) derart ausgebildet ist, dass das fluide Medium zu dem Sensorelement (22) im Wesentlichen mittels Diffusion gelangt.

5. Sensor (10) nach dem vorhergehenden Anspruch, wobei die Blende (52, 56, 64) zum im Wesentlichen Verhindern einer Wärmeübertagung von dem Sensorelement (22) auf das fluide Medium ausgebildet ist.

6. Sensor (10) nach einem der vorhergehenden Ansprüche, wobei das Abzweigungselement (28) zum Verringern einer Strömungsgeschwindigkeit des fluiden Mediums (12) beim Abzweigen aus dem Messraum (14) in den Innenraum (30) ausgebildet ist.

7. Sensor (10) nach einem der vorhergehenden Ansprüche, weiterhin umfassend mehrere Blenden (52, 56, 64), wobei die Blenden (52, 56, 64) in einer Strömungsrichtung von dem Messraum (14) zu dem Sensorelement (22) gesehen derart hintereinander angeordnet sind, dass konvektive Anteile des fluiden Mediums (12) in der Strömungsrichtung gesehen abnehmen.

8. Sensor (10) nach einem der vorhergehenden Ansprüche, wobei das Abzweigungselement (28) zum im Wesentlichen Verhindern eines Zutritts von Flüssigkeitstropfen und/oder Partikeln zu dem Sensorelement (22) ausgestaltet ist.

9. Sensor (10) nach einem der vorhergehenden Ansprüche, wobei der Messraum (14) ein Strömungsrohr ist, insbesondere ein Abgasrohr (18) einer Brennstoffzelle.

## Claims

1. Sensor (10) for detecting at least one property of a fluid medium (12) in a measuring chamber (14), in particular for detecting an H₂ component in a measurement gas (16), comprising:
at least one sensor element (22) designed to detect a thermal conductivity of the fluid medium (12) and to output a measurement signal, the sensor (10) furthermore
defining a branching element (28) that defines an interior (30), the branching element (28) being designed to branch off a portion of the fluid medium (12) from the measuring chamber (14) into the interior (30),
at least one diaphragm (52, 56, 64), the sensor element (22) being fluid-connected to the interior (30) by means of the diaphragm (52, 56, 64), and an exhaust pipe (12), in which the fluid medium (12) flows in a main direction of flow (20), **characterized in that** the branching element (28) is in the form of a venturi nozzle (34) with a U-shaped outlet pipe (36), the branching element (28) having a flow pipe (38) that extends parallel to the main direction of flow of the fluid medium in the exhaust pipe (18), the flow pipe (38) having a first pipe section (40), facing the main direction of flow (20), with a first cross-sectional area (42) and a second pipe section (44), facing away from the main direction of flow (20), with a second cross-sectional area (46), the first cross-sectional area (42) being larger than the second cross-sectional area (46), the outlet pipe (36) being connected to the flow pipe (38) in the region of a cross-sectional narrowing of the flow pipe (38), the outlet pipe (36) having a first opening, which is fluid-connected to the first pipe section (40), and a second opening, which is fluid-connected to the second pipe section (44).

2. Sensor (10) according to the preceding claim, the diaphragm (52, 56, 64) being designed to reduce turbulence in the fluid medium (12).

3. Sensor (10) according to either of the preceding claims, the diaphragm (52, 56, 64) being designed to reduce convective portions of the fluid medium (12).

4. Sensor (10) according to one of the preceding claims, the diaphragm (52, 56, 64) being designed in such a way that the fluid medium reaches the sensor element (22) substantially by means of diffusion.

5. Sensor (10) according to the preceding claim, the diaphragm (52, 56, 64) being designed to substantially prevent a transfer of heat from the sensor element (22) to the fluid medium.

6. Sensor (10) according to one of the preceding claims, the branching element (28) being designed to reduce a flow velocity of the fluid medium (12) when branching it off from the measuring chamber (14) into the interior (30).

7. Sensor (10) according to one of the preceding claims, furthermore comprising multiple diaphragms (52, 56, 64), the diaphragms (52, 56, 64) being arranged in succession in a direction of flow as seen from the measuring chamber (14) to the sensor element (22), in such a way that convective components of the fluid medium (12) as seen in the direction of flow decrease.

8. Sensor (10) according to one of the preceding claims, the branching element (28) being designed to substantially prevent drops of liquid and/or particles from entering the sensor element (22).

9. Sensor (10) according to one of the preceding claims, the measuring chamber (14) being a flow pipe, in particular an exhaust pipe (18) of a fuel cell.

## Revendications

1. Capteur (10) pour la détection d'au moins une propriété d'un milieu fluide (12) dans une chambre de mesure (14), en particulier pour la détection d'une proportion de H₂ dans un gaz de mesure (16), comprenant :
au moins un élément capteur (22), qui est conçu pour détecter une conductivité thermique du milieu fluide (12) et pour émettre un signal de mesure, le capteur (10) présentant en outre
un élément de déviation (28), qui définit une chambre interne (30), l'élément de déviation (28) étant conçu pour dévier une partie du milieu fluide (12) à partir de la chambre de mesure (14) dans la chambre interne (30).
au moins un diaphragme (52, 56, 64), l'élément capteur (22) étant relié fluidiquement à la chambre interne (30) au moyen du diaphragme (52, 56, 64), et un tube d'échappement (12), dans lequel s'écoule le milieu fluide (12) dans un sens d'écoulement principal (20), **caractérisé en ce que** l'élément de déviation (28) est conçu comme un venturi (34) présentant un tube de prélèvement (36) en forme de U, l'élément de déviation (28) présentant un tube d'écoulement (38) qui s'étend parallèlement au sens d'écoulement principal du milieu fluide dans le tube d'échappement (18), le tube d'écoulement (38) présentant une première section tubulaire (40), orientée dans le sens d'écoulement principal (20) et présentant une première surface transversale (42), et une deuxième section tubulaire (44), orientée à l'opposé du sens d'écoulement principal (20) et présentant une deuxième surface transversale (46), la première surface transversale (42) étant supérieure à la deuxième surface transversale (46), le tube de prélèvement (36) étant relié au tube d'écoulement (38) dans la zone d'un rétrécissement transversal du tube d'écoulement (38), le tube de prélèvement (36) présentant une première ouverture, qui est reliée fluidiquement à la première section tubulaire (40), et une deuxième ouverture, qui est reliée fluidiquement à la deuxième section tubulaire (44).

2. Capteur (10) selon la revendication précédente, le diaphragme (52, 56, 64) étant conçu pour réduire les turbulences du milieu fluide (12).

3. Capteur (10) selon la revendication précédente, le diaphragme (52, 56, 64) étant conçu pour réduire les éléments convectifs du milieu fluide (12).

4. Capteur (10) selon l'une des revendications précédentes, le diaphragme (52, 56, 64) étant conçu de telle sorte que le milieu fluide atteint l'élément capteur (22) sensiblement par diffusion.

5. Capteur (10) selon la revendication précédente, le diaphragme (52, 56, 64) étant conçu pour empêcher sensiblement un transfert de chaleur à partir de l'élément capteur (22) au milieu fluide.

6. Capteur (10) selon l'une des revendications précédentes, l'élément de déviation (28) étant conçu pour réduire une vitesse d'écoulement du milieu fluide (12) lors de la déviation à partir de la chambre de mesure (14) dans la chambre interne (30).

7. Capteur (10) selon l'une des revendications précédentes, comprenant en outre plusieurs diaphragmes (52, 56, 64, les diaphragmes (52, 56, 64) étant agencés les uns derrière les autres, vus dans un sens d'écoulement, à partir de la chambre de mesure (14) vers l'élément capteur (22), de telle sorte que les éléments convectifs du milieu fluide (12) diminuent, vus dans le sens d'écoulement.

8. Capteur (10) selon l'une des revendications précédentes, l'élément de déviation (28) étant réalisé pour empêcher sensiblement un accès de gouttelettes de liquide et/ou de particules à l'élément capteur (22).

9. Capteur (10) selon l'une des revendications précédentes, la chambre de mesure (14) étant un tube d'écoulement, en particulier un tube d'échappement (18) d'une pile à combustible.
